(19) Europäisches Patentamt
European Patent Office
Office européen des brevets

(11) Publication number: **0 200 307**
**B1**

(12) **EUROPEAN PATENT SPECIFICATION**

(45) Date of publication of patent specification: **20.12.89**

(21) Application number: **86301405.6**

(22) Date of filing: **27.02.86**

(51) Int. Cl.$^4$: **C 07 D 215/56**, C 07 D 401/04, C 07 D 487/10, C 07 D 487/08, C 07 D 451/04

(54) **Improved process for production of quinoline-3-carboxylic acid antibacterial agents.**

(30) Priority: **25.03.85 US 716005**

(43) Date of publication of application:
**05.11.86 Bulletin 86/45**

(45) Publication of the grant of the patent:
**20.12.89 Bulletin 89/51**

(84) Designated Contracting States:
**AT BE CH DE FR GB IT LI LU NL SE**

(56) References cited:
**EP-A-0 106 489**
**EP-A-0 126 355**
**US-A-4 292 317**

(73) Proprietor: **WARNER-LAMBERT COMPANY**
**201 Tabor Road**
**Morris Plains New Jersey 07950 (US)**

(72) Inventor: **Domagala, John M.**
**776 Georgetown**
**Canton Michigan 48188 (US)**
Inventor: **Schroeder, Mel C.**
**3020 Inverness**
**Dexter Michigan 48130 (US)**

(74) Representative: **Jones, Michael Raymond et al**
**HASELTINE LAKE & CO. Hazlitt House 28**
**Southampton Buildings Chancery Lane**
**London WC2A 1AT (GB)**

**EP 0 200 307 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Belgium Patent 899,399 describes certain 7-piperazine-1-cyclopropyl-6,8-difluoro-1,4-dihydro-4-oxo-3-quinolinecarboxylic acids. German Offenlegungschrift 3318145 describes various 7-amino-1-cyclopropyl-6,8-difluoro-1,4-dihydro-4-oxo-3-quinolinecarboxylic acids. European Patent Publication 106489 describes 7-cyclic amine-1-cyclopropyl-6,8-difluoro-1,4-dihydro-4-oxo-3-quinolinecarboxylic acids.

All of the above compounds are useful as antibacterial agents and have been described as being prepared by displacement of a 7-fluoro atom from a compound of the formula

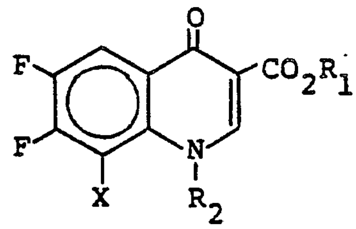

(II)

wherein X is hydrogen or fluorine; $R_1$ is hydrogen or lower alkyl, and $R_2$ is alkyl of one to three carbon atoms or cycloalkyl of three to six carbon atoms, with the appropriate amine.

The object of the present invention is an improved process for preparing the compounds described above by converting a lower alkyl ester of 1-alkyl or cycloalkyl-6,7,8-trifluoro or 6,7-difluoro-1,4-dihydro-4-oxo-3-quinolinecarboxylic acid to the corresponding alkali metal salt and after dissolving the salt formed in situ carrying out the displacement reaction with the amine displacing the 7-fluoro to give the final product. The present method provides better quality material with fewer purification procedures, step-saving by being able to carry out the reaction in one-pot, and higher overall yields from the 1-alkyl or 1-cycloalkyl-6,7,8-trifluoro or 6,7-difluoro-1,4-dihydro-4-oxo-3-quinolinecarboxylic acid alkyl ester.

According to the present invention, there is provided a process for the preparation of compounds of the formula:

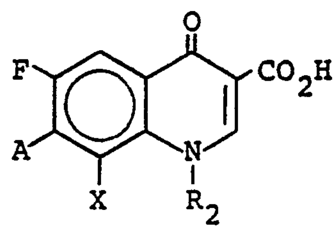

(I)

and pharmaceutically acceptable acid addition or base salts thereof, wherein A is a substituted amino group selected from (i) a mono- or dialkylamino group in which the alkyl portion has one to four carbon atoms and is straight or branched, which alkyl portion is optionally substituted by hydroxy, amino, methylamino or dimethylamino; (ii) a five- or six-membered heterocyclic amino group, which ring is optionally interrupted by another heteroatom and which ring is optionally substituted by alkyl having one to three carbon atoms, hydroxy, alkoxy having one to three carbon atoms, amino, methylamino, ethylamino, aminomethyl, aminoethyl, alkylaminoethyl in which the alkyl has one to three carbon atoms, or alkylaminomethyl in which the alkyl has one to three carbon atoms; (iii) a group of the formula

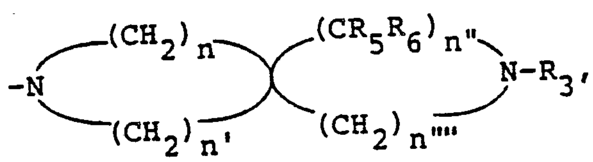

or

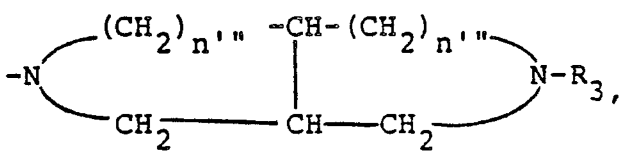

wherein $R_3$ is hydrogen, alkyl having one to four carbon atoms or cycloalkyl having three to six carbon atoms, n is 1, 2, 3 or 4; n' is 1, 2, 3 or 4; n + n' is a total of 2, 3, 4 or 5; n'' is 0, 1 or 2; n''' is 1 or 2; and n'''' is 0, 1 or 2; and (iv) a bicyclic amino group; wherein X is hydrogen or fluorine; and wherein $R_2$ is alkyl having one to three carbon atoms or cycloalkyl having three to six carbon atoms, which process comprises:

(a) reacting 1.0—3.0 equivalent or an alkali metal nucleophile in an anhydrous inert solvent with a compound of the formula

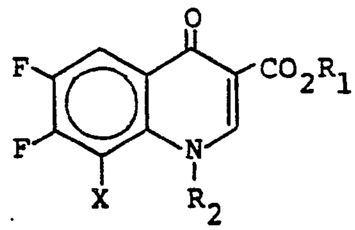

(II)

wherein $R_2$ and X are as defined above and $R_1$ is alkyl having one to three carbon atoms, at 0—150°C to form an alkali metal salt thereof;

(b) adding at least one equivalent of the appropriate amine to the alkali metal salt in an aprotic solvent or in situ to the alkali metal salt solution containing an aprotic co-solvent and heating the reaction mixture between 60 and 120°C until the reaction is complete;

(c) removing the solvent and treating the solid with ester and then with dilute acid to a pH of 5.5—7.5; and

(d) if desired, converting the resulting compound of Formula I to a pharmaceutically acceptable acid addition or base salt thereof;

wherein steps (a), (b) and (c) of the process are carried out as a one-pot process.

The process of the invention has the advantage that, as steps (a), (b) and (c) are carried out as a one-pot process, the process does not need purification steps.

The term "alkyl" in the present invention generally refers to a one to three carbon straight or branched hydrocarbon radical, such as, e.g., methyl, 1- or 2-propyl, and preferably ethyl.

"Cycloalkyl" refers to a three to six-membered saturated hydrocarbon ring such as, e.g., cyclobutyl, cyclopentyl, cyclohexyl, and preferably, cyclopropyl.

When the substituted amino group A is a five- or six-membered heterocyclic amino group interrupted by another heteroatom, the other heteroatom may be, for example, oxygen, sulfur, —SO—, $—SO_2$ or $N—R_3$, wherein $R_3$ is hydrogen, alkyl having one to four carbon atoms or cycloalkyl having three to six carbon atoms.

When the substituted amino group A is a bicyclic amino group, it may be, for example, one selected from

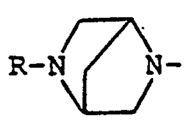 , 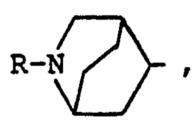 , 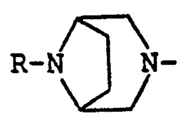 , 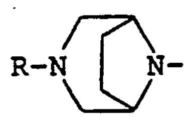 ,

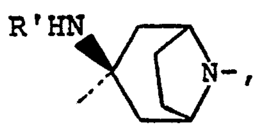 , 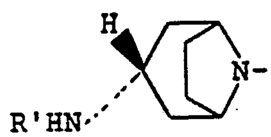 or 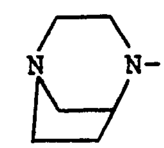

in which R is hydrogen, alkyl of one to three carbon atoms, hydroxylalkyl of two or three carbon atoms, benzyl or p-aminobenzyl, and R′ is hydrogen or alkanoyl of one to three carbon atoms.

Preferred amino groups are piperazine or N-methylpiperazine; a pyrrolidine of the formula

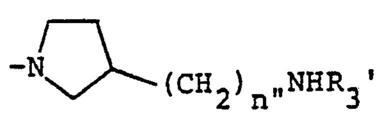

in which n″ is 0 or 1 and $R_3'$ is hydrogen, methyl, ethyl, 1- or 2-propyl; a spiroamine of the formula

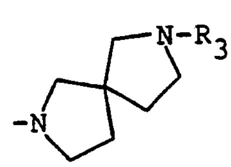

in which $R_3'$ is as defined above, or the above bridged amino groups in which R and R′ are also defined above.

Particularly preferred 7-substituted amino-1-cyclopropyl-6,8-difluoro-1,4-dihydro-4-oxo-3-quinolinecarboxylic acids prepared by the improved process of the present invention are the following:
7-[3-(aminomethyl)-1-pyrrolidinyl]-1-cyclopropyl-6,8-difluoro-1,4-dihydro-4-oxo-3-quinolinecarboxylic acid;
1-cyclopropyl-7-[3-[(ethylamino)methyl]-1-pyrrolidinyl]-6,8-difluoro-1,4-dihydro-4-oxo-3-quinolinecarboxylic acid;
1-ethyl-7-[3-[(ethylamino)methyl]-1-pyrrolidinyl]-6,8-difluoro-1,4-dihydro-4-oxo-3-quinolinecarboxylic acid;
7-[3-amino-1-pyrrolidinyl]-1-cyclopropyl-6,8-difluoro-1,4-dihydro-4-oxo-3-quinolinecarboxylic acid;
1-cyclopropyl-6,8-difluoro-1,4-dihydro-7-[3-[(methylamino)methyl]-1-pyrrolidinyl]-4-oxo-3-quinolinecarboxylic acid;
1-cyclopropyl-7-[3-(ethylamino)-1-pyrrolidinyl]-6,8-difluoro-1,4-dihydro-4-oxo-3-quinolinecarboxylic acid;
1-cyclopropyl-6,8-difluoro-1,4-dihydro-7-[3-[[(1-methylethyl)amino]methyl]-1-pyrrolidinyl]-4-oxo-3-quinolinecarboxylic acid;
7-(2,5-diazabicyclo[2.2.2]oct-2-yl)-1-cyclopropyl-6,8-difluoro-1,4-dihydro-4-oxo-3-quinolinecarboxylic acid;
7-[3-(exo-amino)-8-azabicyclo[3.2.1]oct-8-yl]-1-cyclopropyl-6,8-difluoro-1,4-dihydro-4-oxo-3-quinolinecarboxylic acid;
7-(1,4-diazabicyclo[3.2.1]oct-4-yl)-1-cyclopropyl-6,8-difluoro-1,4-dihydro-4-oxo-3-quinolinecarboxylic acid;
1-cyclopropyl-6,8-difluoro-1,4-dihydro-7-(5-methyl-2,5-diazabicyclo[2.2.1]hept-2-yl)-4-oxo-3-quinolinecarboxylic acid;
1-cyclopropyl-7-[2,5-diazabicyclo(2.2.1)hept-2-yl]-6,8-difluoro-1,4-dihydro-4-oxo-3-quinolinecarboxylic acid,
and the pharmaceutically acceptable acid addition or base salts thereof.

As previously described, the compounds of Formula I are useful as antibacterial agents against both gram-positive and gram-negative bacteria.

An alkali metal nucleophile includes, for example, lithium, sodium or potassium hydroxide, halide, preferably iodide or bromide, or trimethylsilanoate.

The improved process for the preparation of compounds of Formula I proceeds by way of example as follows:

To the appropriate alcohol is added 1.0—3.0 equivalents of alkali metal and the mixture is heated at reflux until formation of the metal alkoxide is complete. A preformed alkali metal alkoxide can be alternatively employed. At room temperature, 1.0—3.0 equivalents of water are added to generate the anhydrous alkali metal hydroxide. This reaction may be carried out in the same alcohol as solvent or in an inert solvent such as dioxane, tetrahydrofuran or diglyme and the like. The mixture is cooled to 0—20°C and 1-cyclopropyl-6,7,8-trifluoro or 6,7-difluoro-1,4-dihydro-4-oxo-3-quinolinecarboxylic acid ethyl or methyl ester is added in the same alcohol as above, in an inert solvent, or neat. The mixture is gradually warmed to 25—80°C. The reaction is continued until the disappearance of the starting ester is complete as determined by thin layer chromatography. The solvents are then removed and the 1-cyclopropyl-6,7,8-trifluoro-1,4-dihydro-4-oxo-3-quinolinecarboxylic acid alkali metal salt is used for the next step. For example a co-solvent such as N,N-dimethylformamide, dimethylsulfoxide, acetonitrile, or other like aprotic solvent is added to the mixture in situ and the mixture used for the next step.

Alternatively, other nucleophiles may be employed for the preparation of alkali salts directly from esters. These include the treatment of a 1-cyclopropyl-6,7,8-trifluoro-1,4-dihydro-4-oxo-3-quinoline carboxylic acid alkyl ester with for example, lithium iodide in an inert solvent including pyridine, collidine, N,N-dimethylformamide, or dimethylsulfoxide at temperatures of 100—150°C; or with sodium or potassium trimethylsilanoate ($NaOSiMe_3$ or $KOSiMe_3$) in ethereal or chlorocarbon solvents. As described above, salts prepared by these methods may be isolated by conventional methods or may be used in situ in the next step.

To the 1-cyclopropyl-6,7,8-trifluoro or 6,7-difluoro-1,4-dihydro-4-oxo-3-quinolinecarboxylic acid alkali metal salt suspended in the appropriate aprotic solvent (i.e., N,N-dimethylformamide, N,N-dimethyl acetamide, dimethylsulfoxide, acetonitrile, etc. or mixture of these) or to the co-solvent mixture containing said alkali metal salt is added at least one, preferably 1.0—2.0 equivalents, of the appropriate amine to be coupled to the $C_7$ position. The mixture is heated at 60—120°C until the reaction is complete as analyzed by thin layer chromatography. The solvents are removed and the residue is mixed with water. The solution or suspension which is between pH 3—11.5 is then acidified with dilute acid, e.g., 0.3—2N hydrochloric or sulfuric acids or dilute acetic acid, to a pH 5.5—7.5. The solids are filtered, washed with inert solvents of choice and dried to give the desired 7-substituted amino-1-cyclopropyl-6,8-difluoro-1,4-dihydro-4-oxo-3-quinolinecarboxylic acid.

The choice of alkyl ester, alkoxide, and alcohol solvent preferably conform for the particular synthetic sequence and preferably is made from an alkyl part having a carbon chain of one to three carbon atoms including straight and branched, such as methyl, ethyl, n-propyl or isopropyl, corresponding alkoxides and alcohols.

Alkali metals are preferably lithium, sodium, or potassium.

The starting materials for the present invention are known, or if new, may be prepared from known starting materials. Thus, ethyl 1-cyclopropyl-6,7,8-trifluoro-1,4-dihydro-4-oxo-3-quinolinecarboxylate is prepared as described in Belgian Patent 899,399 or DE3318145. Alternatively, it may be prepared by a series of reactions starting from 2,3,4,5-tetrafluorobenzoic acid. The sodium salt of 2,3,4,5-tetrafluorobenzoic acid is reacted with oxalyl chloride and the product condensed with diethyl malonate in the presence of magnesium turnings to afford after hydrolysis 2,3,4,5-tetrafluorobenzoylacetic acid, ethyl ester. This compound is, in turn, treated with triethylorthoformate and acetic anhydride, followed by cyclopropylamine to afford 2-(2,3,4,5-tetrafluorobenzoyl)-3-cyclopropylamino acrylic acid, ethyl ester, which is then ring closed to give the desired starting material. The corresponding methyl ester is prepared using dimethylmalonate in the sequence described above.

The substituted amines used herein are either known compounds or they may be prepared from known starting materials by standard procedures or by variations thereof. For example, 3-pyrrolidine-methanamines having the structural formula D

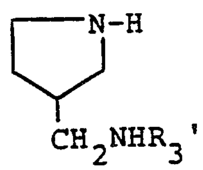

D

may be readily prepared from the known starting material methyl 5-oxo-1-(phenylmethyl)-3-pyrrolidine-carboxylate, A, [J. Org. Chem., *26*, 1519 (1961)] by the following reaction sequence.

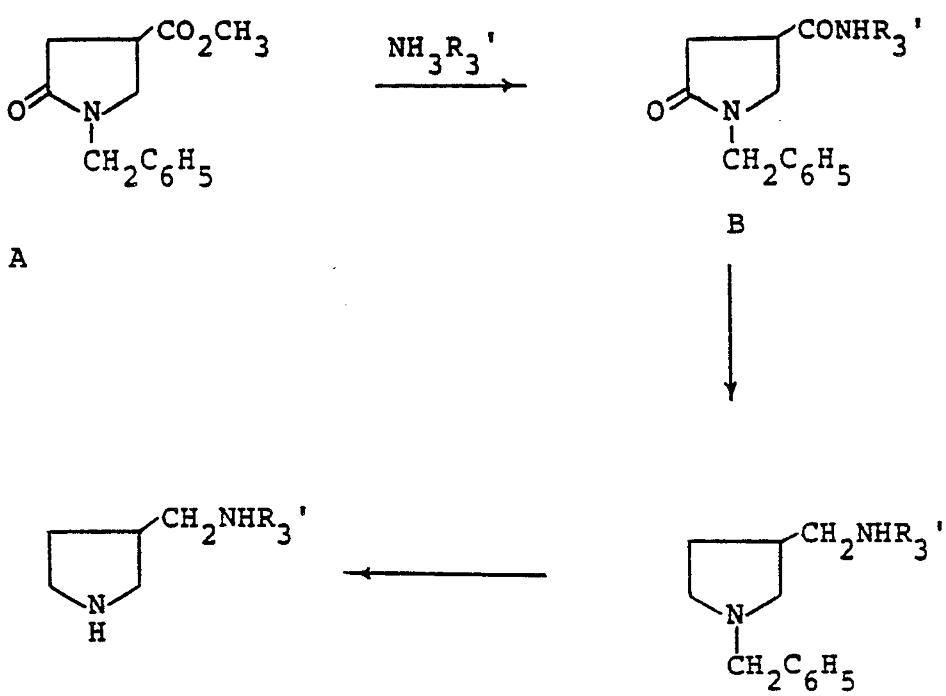


A

B

D

C

The compound wherein $R_3'$ is hydrogen, namely 3-pyrrolidinemethanamine, has been reported in J. Org. Chem., *26*, 4955 (1961).

Thus Compound A may be converted to the corresponding amide B by treatment with $R_3'NH_2$; for example, a saturated solution of ethylamine in an alkanol such as methyl alcohol may be utilized. The diamide B may next be reduced to produce the corresponding diamine C. This reduction may be carried out using lithium aluminum hydride, for example, in a convenient solvent such as tetrahydrofuran. Compound

C may next be debenzylated, for example using hydrogen and 20% palladium on carbon catalyst to produce the diamine D. Alternatively, when $R_3$ = H in C, the primary amine function may be protected by acylation with an acyl halide such as acetyl chloride by well known procedures. The primary amine function of C may also be converted to a carbamate ester such as the ethyl ester by treatment with ethyl chloroformate in the presence of a base such as 1,8-diazabicyclo[5.4.0]undec-7-ene in a convenient solvent such as methylene chloride. The benzyl group may next be removed, for example as described above for Compound C, thereby producing Compound D where $R_3$ is $—CO_2Et$, which after conversion to a compound of the type VIa or VIb may be reacted with a compound having the structural formula IV or V to thereby produce a corresponding compound having the structural formulae I or Ia. The $—CO_2Et$ group may be removed by standard procedures.

Likewise spiroamino compounds may be readily prepared from the known starting material 3-ethoxycarbonyl-5-oxo-3-pyrrolidineacetic acid ethyl ester [J. Org. Chem., 46, 2757 (1981)] by the following reaction sequence.

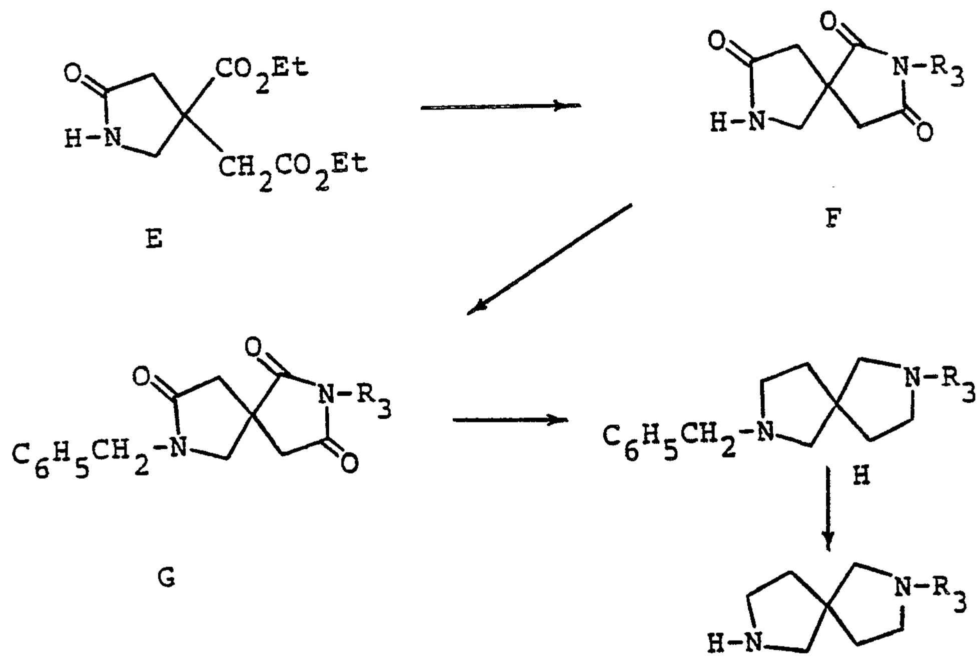

E F G H J

The compound 2,7-diazaspiro[4.4]nonane where $R_3$ is H is described in the above reference. Thus Compound E may be converted to the corresponding amide F by treatment with $R_3NH_2$, for example, methyl amine in water followed by benzylation which may be carried out with sodium hydride and benzyl chloride to give G. Reduction to the diamine H may be accomplished with lithium aluminum hydride. Subsequent debenzylation, for example, with hydrogen and 20% palladium on carbon catalyst produces the diamine J.

The bridged amino compounds are either known compounds or they may be prepared from known starting materials by standard procedures or by variations thereof. For example, exo and endo 3-amino-8-azabicyclo[3.2.1]octanes having the structural formula B and the acetyl derivatives E

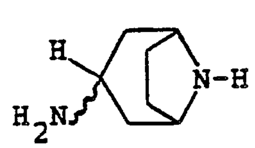

B

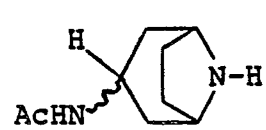

E

may be readily prepared from the known starting material 8-(phenylmethyl)-8-azabicyclo[3.2.1]octan-3-one oxime, A, [J. Heterocyclic Chem., *19*, 485 (1982)] by the following reaction sequence.

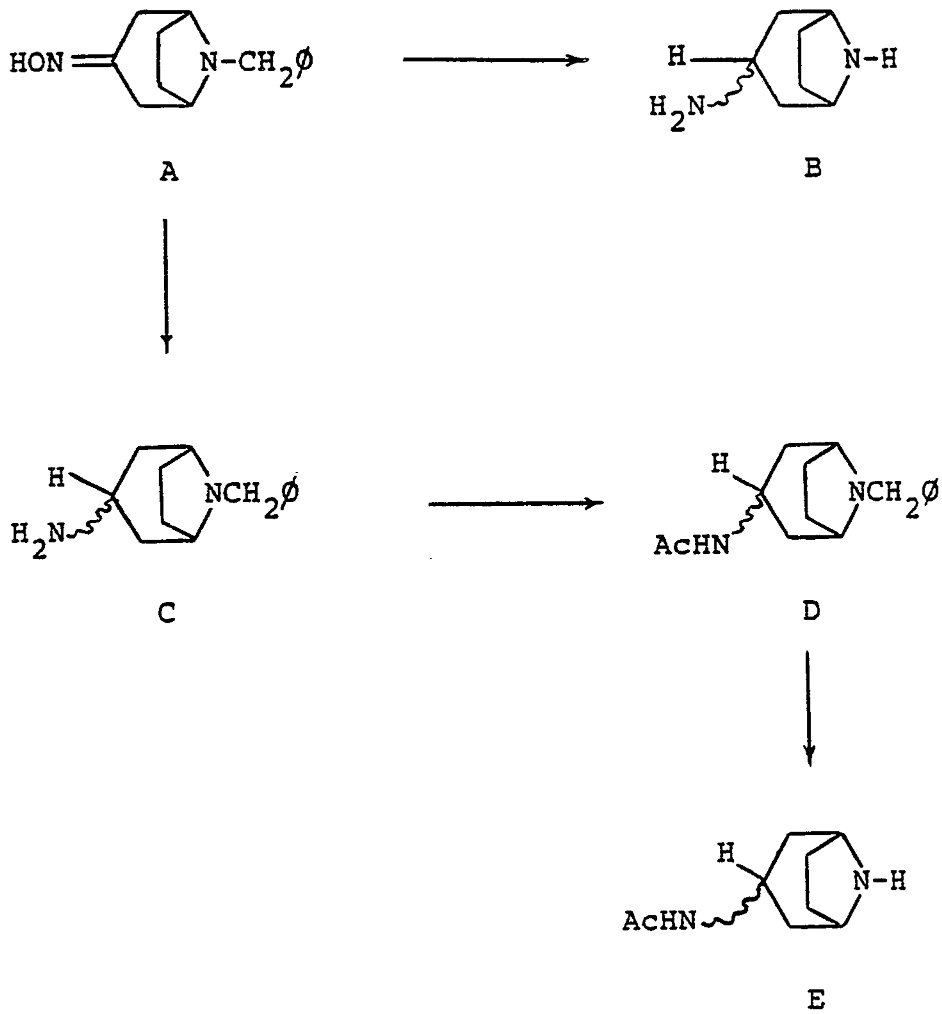

The compounds prepared by the present invention are capable of further forming both pharmaceutically acceptable acid addition and/or base salts. Base salts are formed with metals or amines, such as alkali and alkaline earth metals or organic amines. Examples of metals used as cations are sodium, potassium, magnesium, calcium, and the like. Also included are heavy metal salts such as for example silver, zinc, cobalt, and cerium. Such heavy metal salts are effective in the treatment of burns especially when applied to the affected surface of a burn victim either directly or in combination with a physiologically acceptable carrier such as a water dispersible, hydrophilic carrier. Examples of suitable amines are N,N'dibenzylethylenediamine, chloroprocaine, choline, diethanolamine, ethylenediamine, N-methylglucamine, and procaine.

Pharmaceutically acceptable acid addition salts are formed with organic and inorganic acids.

Examples of suitable acids for salt formation are hydrochloric, sulfuric, phosphoric, acetic, citric, oxalic, malonic, salicylic, malic, gluconic, fumaric, succinic, ascorbic, maleic, methanesulfonic, and the like. The salts are prepared by contacting the free base form with a sufficient amount of the desired acid to produce either a mono or di, etc salt in the conventional manner. The free base forms may be regenerated by treating the salt form with a base. For example, dilute solutions of aqueous base may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention. Use of excess base where R' is hydrogen gives the corresponding basic salt.

The compounds of the invention can exist in unsolvated as well as solvated forms, including hydrated forms. In general, the solvated forms, including hydrated forms and the like are equivalent to the unsolvated forms for purposes of the invention.

Certain compounds of the invention may exist in optically active forms. The pure D isomer, pure L isomer as well as mixtures thereof; including the racemic mixtures, are contemplated by the invention. Additional assymmetric carbon atoms may be present in a substituent such as an alkyl group. All such isomers as well as mixtures thereof are intended to be included in the invention.

The following nonlimiting examples illustrate the inventors' preferred methods for preparing the compounds of the invention.

Example 1

1-Cyclopropyl-6,7,8-trifluoro-1,4-dihydro-4-oxo-3-quinolinecarboxylic acid, sodium salt

Method A: To a mixture of 111 mg (0.0048 g·atom) of sodium metal dissolved in 2 ml of ethanol and 15 ml of dioxane was added at 25°C 87 mg (4.8 mmol) of water. After 15 minutes, the mixture was cooled to 10°C and 1.0 g (3.2 mmol) of 1-cyclopropyl-6,7,8-trifluoro-1,4-dihydro-4-oxo-3-quinolinecarboxylic acid ethyl ester was added slurried in 5 ml of dioxane. The mixture was stirred at 20°C for 15 minutes and the temperature was gradually raised to 35°C. Analysis by thin layer chromatography showed that the reaction was complete after two hours. The solvent was removed under vacuum to give 0.72 g (75%) of the 1-cyclopropyl-6,7,8-trifluoro-1,4-dihydro-4-oxo-3-quinolinecarboxylic acid, sodium salt, mp >285°C (with gradual decomposition).

Method B: To 1.0 g (3.2 mmol) of the 1-cyclopropyl 6,7,8-trifluoro-1,4-dihydro-4-oxo-3-quinolinecarboxylic acid ethyl ester in chloroform was added 0.43 g (1.2 equivalents) of sodium trimethylsilanoate. The reaction was refluxed for two hours and was concentrated. The solids were washed with ether and collected to give 0.98 g (100%) of the title compound mp >290°C (with gradual decomposition).

Example 2

1-Cyclopropyl-6,7,8-trifluoro-1,4-dihydro-4-oxo-3-quinolinecarboxylic acid, potassium salt

Method A: To a mixture of 250 mg (0.0064 g·atom) of potassium metal dissolved in 5 ml of methanol and 25 ml of dioxane was added, at 25°C, 116 mg (6.4 mmol) of water. After 15 minutes, the mixture was cooled to 0°C and 0.95 g (3.2 mmol) of 1-cyclopropyl-6,7,8-trifluoro-1,4-dihydro-4-oxo-3-quinolinecarboxylic acid methyl ester was added as a solid. The mixture was stirred at 0°C for 15 minutes and was gradually warmed to 35°C. The reaction was complete after two hours. The solvent was removed under vacuum to give 0.78 g (75%) of the 1-cyclopropyl-6,7,8-trifluoro-1,4-dihydro-4-oxo-3-quinolinecarboxylic acid, potassium salt, mp >275°C (with gradual decomposition).

Method B: To 1.0 g (3.2 mmol) of 1-cyclopropyl-6,7,8-trifluoro-1,4-dihydro-4-oxo-3-quinoline carboxylic acid ethyl ester in 20 ml of dichloromethane was added 0.48 g (3.7 mmol) of potassium trimethylsilanoate. After 2.5 hours at reflux, an additional 0.48 g of potassium trimethylsilanoate was added. The reaction was continued an additional two hours. The precipitate was filtered and washed with ether to give 0.96 g (93%) of the crude title compound, mp >300°C.

Example 3

Preparation of 1-cyclopropyl-7-[3-[(ethylamino)methyl]-1-pyrrolidinyl]-6,8-difluoro-1,4-dihydro-4-oxo-3-quinolinecarboxylic acid

Method A: To 150 mg (0.47 mmol) of the 1-cyclopropyl-6,7,8-trifluoro-1,4-dihydro-4-oxo-3-quinolinecarboxylic, sodium salt suspended in 15 ml of dry acetonitrile was added 76 mg (1.2 equivalents) of N-ethyl-3-pyrrolidinemethanamine in 3.5 ml of additional acetonitrile. The reaction was refluxed for one hour and 5 ml of N,N-dimethylformamide was added. The reaction was refluxed an additional 8.5 hours and was concentrated. The residue was mixed with water to give a light suspension at pH 11.0. The mixture was treated with dilute hydrochloric acid to a pH 6.8. The thick solids were filtered, washed with ether, and dried to give 170 mg (88%) of the 1-cyclopropyl-7-[3-[(ethylamino)methyl]-1-pyrrolidinyl]-6,8-difluoro-1,4-dihydro-4-oxo-3-quinolinecarboxylic acid, mp 256—258°C. Using the identical conditions with the 1-cyclopropyl-6,7,8-trifluoro-1,4-dihydro-4-oxo-3-quinolinecarboxylic acid potassium salt gave 149 mg (81%) of the title compound, mp 256—258°C.

Method B: Alternatively the following procedure may be employed. To a mixture of 100 mg (0.0043 g·atom) of sodium metal dissolved in 2 ml of methanol and 12 ml of dioxane was added, at 25°C, 77 mg (1.0 equivalent) of water. After 15 minutes 0.95 g (3.2 mmol) of 1-cyclopropyl-6,7,8-trifluoro-1,4-dihydro-4-oxo-3-quinolinecarboxylic acid, methyl ester was added as a dry powder. The mixture was stirred for one hour at 20°C and gradually warmed to 30°C. After ten hours, the mixture was treated with 0.49 g (1.2 equivalents) of N-ethyl-3-pyrrolidinemethanamine in 25 ml of acetonitrile. The mixture was refluxed for 24 hours. The solvents were removed, under vacuum, and the solids mixed with water. The pH was adjusted to 7.0 and the solids collected, washed with ether and dried to give 0.90 g (72%) of the title compound, mp 256—258°C.

Example 4

Preparation of 7-[3-amino-1-pyrrolidinyl]-1-cyclopropyl-6,8-difluoro-1,4-dihydro-3-quinolinecarboxylic acid

To 300 mg (0.98 mmol) of the 1-cyclopropyl-6,7,8-trifluoro-1,4-dihydro-4-oxo-3-quinolinecarboxylic acid, sodium salt, suspended in 20 ml of acetonitrile was added 236 mg (1.3 equivalents) of 3-butoxycarbonylaminopyrrolidine in 5 ml of N,N-dimethylformamide. The mixture was heated at 90°C for nine hours. It was concentrated and the residue mixed with water. The mixture was acidified with 2N hydrochloric acid and refluxed for two hours. The pH was adjusted to pH of 5.5 and the solids were filtered, washed with ether and dried to give 300 mg (87%) of the title compound, mp 290—292°.

Example 5

1-Cyclopropyl-7-(2,5-diazabicyclo[2.2.1]hept-2-yl)-6,8-difluoro-1,4-dihydro-4-oxo-3-quinolinecarboxylic acid

To 150 mg (0.47 mmol) of the 1-cyclopropyl-6,7,8-trifluoro-1,4-dihydro-4-oxo-3-quinolinecarboxylic acid sodium salt, suspended in 15 ml of acetonitrile was added 162 mg (1.3 equivalents) of 2,5-diazabicyclo[2.2.1]heptane dihydrobromide and 186 mg (2.0 equivalents) of 1,8-diazabicyclo[5.4.0]undec-7-ene in 5 ml of N,N-dimethylformamide. The mixture was heated for ten hours at 100°C. It was concentrated and the residue mixed with water. The mixture was acidified with dilute acid to pH 7.0 and the solids were isolated, washed with ether and dried to give 149 mg (88%) of the title compound, >250°C mp.

## Claims

1. A process for the preparation of compounds of the formula:

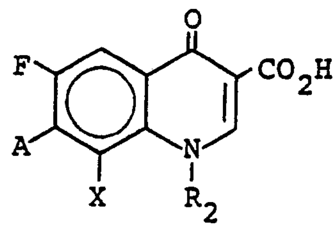

(I)

and pharmaceutically acceptable acid addition or base salts thereof, wherein A is a substituted amino group selected from (i) a mono- or dialkylamino group in which the alkyl portion has one to four carbon atoms and is straight or branched, which alkyl portion is optionally substituted by hydroxy, amino, methylamino or dimethylamino; (ii) a five- or six-membered heterocyclic amino group, which ring is optionally interrupted by another heteroatom and which ring is optionally substituted by alkyl having one to three carbon atoms, hydroxy, alkoxy having one to three carbon atoms, amino, methylamino, ethylamino, aminomethyl, aminoethyl, alkylaminoethyl in which the alkyl has one to three carbon atoms, or alkylaminomethyl in which the alkyl has one to three carbon atoms; (iii) a group of the formula

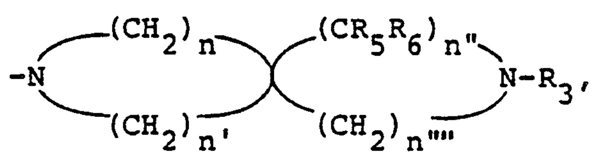

or

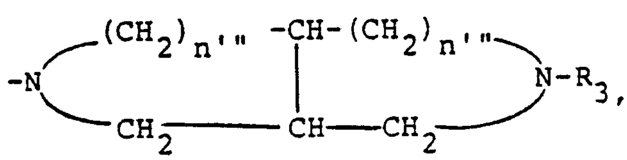

wherein $R_3$ is hydrogen, alkyl having one to four carbon atoms or cycloalkyl having three to six carbon atoms, n is 1, 2, 3 or 4; n' is 1, 2, 3 or 4; n + n' is a total of 2, 3, 4 or 5; n'' is 0, 1 or 2; n''' is 1 or 2; and n'''' is 0, 1 or 2; and (iv) a bicyclic amino group; wherein X is hydrogen or fluorine; and wherein $R_2$ is alkyl having one to three carbon atoms or cycloalkyl having three to six carbon atoms, which process comprises:

(a) reacting 1.0—3.0 equivalent or an alkali metal nucleophile in an anhydrous inert solvent with a compound of the formula

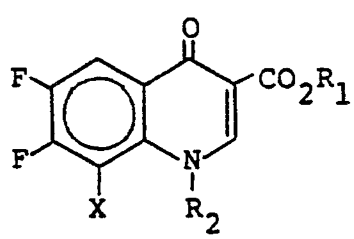

(II)

wherein $R_2$ and X are as defined above and $R_1$ is alkyl having one to three carbon atoms, at 0—150°C to form an alkali metal salt thereof;

(b) adding at least one equivalent of the appropriate amine to the alkali metal salt in an aprotic solvent or in situ to the alkali metal salt solution containing an aprotic co-solvent and heating the reaction mixture between 60 and 120°C until the reaction is complete;

(c) removing the solvent and treating the solid with ester and then with dilute acid to a pH of 5.5—7.5; and

(d) if desired, converting the resulting compound of Formula I to a pharmaceutically acceptable acid addition or base salt thereof;

wherein steps (a), (b) and (c) of the process are carried out as a one-pot process.

2. A process according to claim 1, wherein in step (a) the anhydrous inert solvent is selected from methanol, ethanol, isopropanol, dioxane, tetrahydrofuran, diglyme, pyridine, collidine, dichloromethane, or chloroform.

3. A process according to claim 1 or 2, wherein the methyl or ethyl ester of 1-ethyl or 1-cyclopropyl-6,7,8-trifluoro-1,4-dihydro-4-oxo-3-quinolinecarboxylic acid is used as starting material.

4. A process according to claim 1, 2 or 3 wherein the alkali metal nucleophile is an alkali metal hydroxide, halide, or trimethylsilanoate.

5. A process according to claim 4, wherein the alkali metal hydroxide is sodium or potassium.

6. A process according to claim 4, wherein the alkali metal nucleophile is lithium iodide, lithium bromide, sodium trimethylsilyloxide or potassium trimethylsilanoate.

7. A process according to claim 1, wherein the step (b) the aprotic solvent is selected from N,N-dimethylformamide, N,N-dimethylacetamide, dimethylsulfoxide, and acetonitrile.

8. A process according to any preceding claim, wherein A is said five- or six-membered heterocyclic amino group, and wherein said another heteroatom is selected from oxygen, sulfur, —SO—, —$SO_2$ and N—$R_3$, wherein $R_3$ is as defined in claim 1.

9. A process according to any of claims 1 to 7, wherein A is said five- or six-membered heterocyclic amino group, which ring is optionally interrupted by N—$R_3$ and which ring is optionally substituted by alkyl having one to three carbon atoms, amino, methylamino, ethylamino, alkylaminoethyl in which the alkyl has one to three carbon atoms, or alkylaminomethyl in which the alkyl has one to three carbon atoms, wherein $R_3$ is hydrogen or alkyl having one to four carbon atoms.

10. A process according to any of claims 1 to 7, wherein A is piperazine, N-methylpiperazine or pyrrolidine of the formula

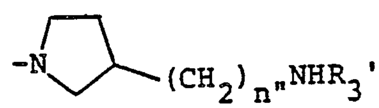

in which n'' is 0 or 1 and $R_3'$ is hydrogen, methyl, ethyl, 1- or 2-propyl.

11. A process according to any of claims 1 to 7, wherein A is of the formula

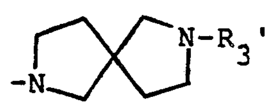

in which $R_3'$ is hydrogen, methyl, ethyl, 1- or 2-propyl.

12. A process according to any of Claims 1 to 7, wherein A is of the formula

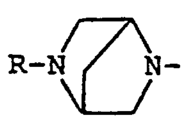 , 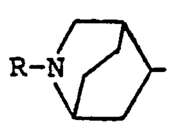 , 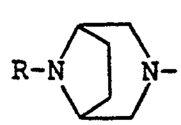 , 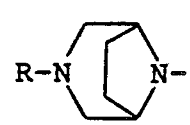 ,

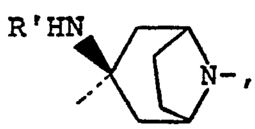 , 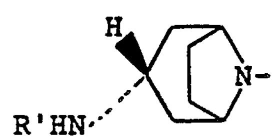 or 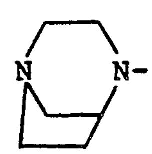

in which R is hydrogen, methyl, ethyl, 1- or 2-propyl, hydroxyethyl, benzyl, or p-aminobenzyl, and R' is hydrogen or acetyl.

13. A process according to any of Claims 1 to 7 in which one of the following compound is prepared:

7-[3-(aminomethyl)-1-pyrrolidinyl]-1-cyclopropyl-6,8-difluoro-1,4-dihydro-4-oxo-3-quinolinecarboxylic acid;

1-cyclopropyl-7-[3-[(ethylamino)methyl]-1-pyrrolidinyl]-6,8-difluoro-1,4-dihydro-4-oxo-3-quinolinecarboxylic acid;

7-[3-amino-1-pyrrolidinyl]-1-cyclopropyl-6,8-difluoro-1,4-dihydro-4-oxo-3-quinolinecarboxylic acid;

1-cyclopropyl-6,8-difluoro-1,4-dihydro-7-[3-[(methylamino)methyl]-1-pyrrolidinyl]-4-oxo-3-quinolinecarboxylic acid;

1-cyclopropyl-7-[3-(ethylamino)-1-pyrrolidinyl]-6,8-difluoro-1,4-dihydro-4-oxo-3-quinolinecarboxylic acid;

1-cyclopropyl-6,8-difluoro-1,4-dihydro-7-[3-[[(1-methylethyl)amino]methyl]-1-pyrrolidinyl]-4-oxo-3-quinolinecarboxylic acid;

7-(2,5-diazabicyclo[2.2.2]oct-2-yl)-1-cyclopropyl-6,8-difluoro-1,4-dihydro-4-oxo-3-quinolinecarboxylic acid;

7-[3-(exo-amino)-8-azabicyclo[3.2.1]oct-8-yl]-1-cyclopropyl-6,8-difluoro-1,4-dihydro-4-oxo-3-quinolinecarboxylic acid;

7-(1,4-diazabicyclo[3.2.1]oct-4-yl)-1-cyclopropyl-6,8-difluoro-1,4-dihydro-4-oxo-3-quinolinecarboxylic acid;

1-cyclopropyl-6,8-difluoro-1,4-dihydro-7-(5-methyl-2,5-diazabicyclo[2.2.1]hept-2-yl)-4-oxo-3-quinolinecarboxylic acid;

1-cyclopropyl-7-(2,5-diazabicyclo[2.2.1]hept-2-yl)-6,8-difluoro-1,4-dihydro-4-oxo-3-quinolinecarboxylic acid; or

1-ethyl-7-[3-[(ethylamino)methyl]-1-pyrrolidinyl]-6,8-difluoro-1,4-dihydro-4-oxo-3-quinolinecarboxylic acid.

**Patentansprüche**

1. Verfahren zur Herstellung von Verbindungen der Formel

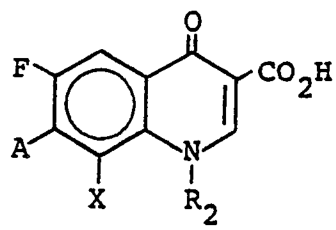

(I)

und pharmazeutisch akzeptabler Säureadditions- oder basischer Salze davon, worin A eine substituierte Aminogruppe ist, ausgewählt aus (i) einer Mono- oder Dialkylaminogruppe, in der der Alkylteil ein bis vier Kohlenstoffatome besitzt und gerade oder verzweigt ist, welcher Alkylteil gegebenenfalls durch Hydroxy, Amino, Methylamino oder Dimethylamino substituiert ist; (ii) einer fünf- oder sechsgliedrigen heterocyclischen Aminogruppe, welcher Ring gegebenenfalls durch ein weiteres Heteroatom unterbrochen ist und welcher Ring gegebenenfalls durch Alkyl mit einem bis drei Kohlenstoffatomen, Hydroxy, Alkoxy mit einem bis drei Kohlenstoffatomen, Amino, Methylamino, Ethylamino, Aminomethyl, Aminoethyl, Alkylaminoethyl, worin das Alkyl ein bis drei Kohlenstoffatome besitzt, oder Alkylaminomethyl, worin das Alkyl ein bis drei Kohlenstoffatome besitzt, substituiert ist; (iii) einer Gruppe der Formel

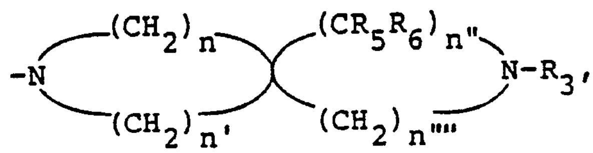

oder

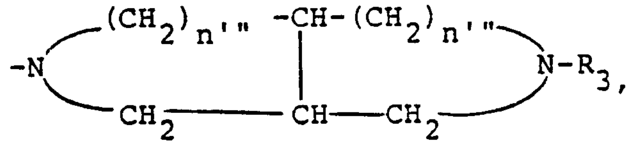

worin $R_3$ Wasserstoff, Alkyl mit einem bis vier Kohlenstoffatomen oder Cycloalkyl mit drei bis sechs Kohlenstoffatomen darstellt, n 1, 2, 3 oder 4 ist; n' 1, 2, 3 oder 4 ist; n + n' zusammen 2, 3, 4 oder 5 ergeben; n'' 0, 1 oder 2 ist; n''' 1 oder 2 ist; und n'''' 0, 1 oder 2 ist; und (iv) einer bicyclischen Aminogruppe; worin X Wasserstoff oder Fluor darstellt; und worin $R_2$ Alkyl mit einem bis drei Kohlenstoffatomen oder Cycloalkyl mit drei bis sechs Kohlenstoffatomen bedeutet, welches Verfahren umfaßt:

a) die Umsetzung von 1,0—3,0 Aquivalenten eines Alkalimetallnukleophils in einem wasserfreien inerten Lösungsmittel mit einer Verbindung der Formel

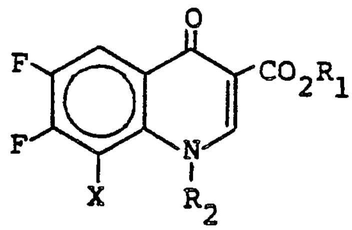

(II)

worin $R_2$ und X wie oben definiert sind und $R_1$ Alkyl mit einem bis drei Kohlenstoffatomen darstellt, bei 0 bis 150°C zur Bildung eines Alkalimetallsalzes davon;

b) die Zugabe von zumindest einem Äquivalent des geeigneten Amins zum Alkalimetallsalz in einem aprotischen Lösungsmittel oder in situ zur Alkalimetallsalzlösung, enthaltend ein aprotisches Co-Lösungsmittel, und Erhitzen der Reaktionsmischung auf zwischen 60 und 120°C bis zur Beendigung der Reaktion;

c) Entfernung des Lösungsmittels und Behandlung des Feststoffs mit Ester und dann mit verdünnter Säure auf einem pH von 5,5—7,5; und

d) gewünschtenfalls Umwandlung der resultierenden Verbindung der Formel I in ein pharmazeutisch akzeptables Säureadditions- oder basisches Salz davon;

wobei die Stufen a), b) und c) des Verfahrens als Eintopfverfahren ausgeführt werden.

2. Verfahren nach Anspruch 1, worin in Stufe a) das wasserfreie inerte Lösungsmittel ausgewählt ist aus Methanol, Ethanol, Isopropanol, Dioxan, Tetrahydrofuran, Diglym, Pyridin, Kollidin, Dichlormethan oder Chloroform.

3. Verfahren nach Anspruch 1 oder 2, worin der Methyl- oder Ethylester von 1-Ethyl- oder 1-Cyclopropyl-6,7,8-trifluor-1,4-dihydro-4-oxo-3-chinolincarbonsäure als Ausgangsmaterial eingesetzt wird.

4. Verfahren nach Anspruch 1, 2 oder 3, worin das Alkalimetallnukleophil ein Alkalimetallhydroxid, -halogenid oder -trimethylsilanoat ist.

5. Verfahren nach Anspruch 4, worin das Alkalimetallhydroxid Natrium- oder Kaliumhydroxid ist.

6. Verfahren nach Anspruch 4, worin das Alkalimetallnukleophil Lithiumjodid, Lithiumbromid, Natriumtrimethylsilyloxid oder Kaliumtrimethylsilanoat ist.

7. Verfahren nach Anspruch 1, worin in Stufe b) das aprotische Lösungsmittel ausgewählt ist aus N,N-Dimethylformamid, N,N-Dimethylacetamid, Dimethylsulfoxid und Acetonitril.

8. Verfahren nach einem vorhergehenden Anspruch, worin A die fünf- oder sechsgliedrige heterocyclische Aminogruppe ist und worin das andere Heteroatom ausgewählt ist aus Sauerstoff, Schwefel, —SO—, —$SO_2$ und N—$R_3$, wobei $R_3$ wie in Anspruch 1 definiert ist.

9. Verfahren nach einem der Ansprüche 1 bis 7, worin A die fünf- oder sechsgliedrige heterocyclische Aminogruppe ist, welcher Ring gegebenenfalls durch N—$R_3$ unterbrochen ist und welcher Ring gegebenenfalls durch Alkyl mit einem bis drei Kohlenstoffatomen, Amino, Methylamino, Ethylamino, Alkylaminoethyl, worin das Alkyl ein bis drei Kohlenstoffatome besitzt, oder Alkylaminomethyl, worin das Alkyl ein bis drei Kohlenstoffatome besitzt, substituiert ist, wobei $R_3$ Wasserstoff oder Alkyl mit einem bis vier Kohlenstoffatomen ist.

10. Verfahren nach einem der Ansprüche 1 bis 7, worin A Piperazin, N-Methylpiperazin oder Pyrrolidin der Formel

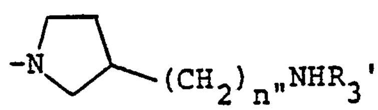

ist, worin n'' 0 oder 1 ist und $R_3'$ für Wasserstoff, Methyl, Ethyl, 1- oder 2-Propyl steht.

11. Verfahren nach einem der Ansprüche 1 bis 7, worin A der Formel

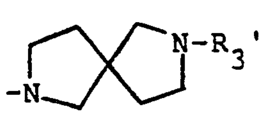

entspricht, in welcher $R_3'$ Wasserstoff, Methyl, Ethyl, 1- oder 2-Propyl ist.

12. Verfahren nach einem der Ansprüche 1 bis 7, worin A der Formel

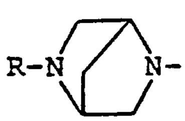 , 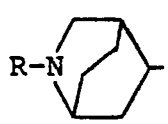 , 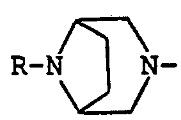 , 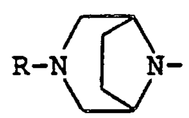 ,

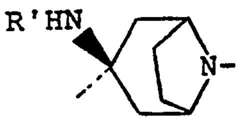

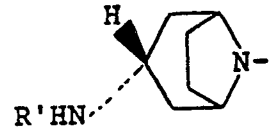

oder

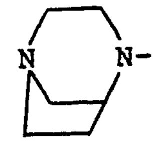

entspricht, in welcher R Wasserstoff, Methyl, Ethyl, 1- oder 2-Propyl, Hydroxyethyl, Benzyl oder p-Aminobenzyl ist und R' für Wasserstoff oder Acetyl steht.

13. Verfahren nach einem der Ansprüche 1 bis 7, bei welchem eine der folgenden Verbindungen hergestellt wird:

7-[3-(Aminomethyl)-1-pyrrolidinyl]-1-cyclopropyl-6,8-difluor-1,4-dihydro-4-oxo-3-chinolincarbonsäure;

1-Cyclopropyl-7-[3-[(ethylamino)-methyl]-1-pyrrolidinyl]-6,8-difluor-1,4-dihydro-4-oxo-3-chinolincarbonsäure;

7-[3-Amino-1-pyrrolidinyl]-1-cyclopropyl-6,8-difluor-1,4-dihydro-4-oxo-3-chinolincarbonsäure;

1-Cyclopropyl-6,8-difluor-1,4-dihydro-7-[3-[(methylamino)methyl]-1-pyrrolidinyl]-4-oxo-3-chinolincarbonsäure;

1-Cyclopropyl-7-[3-(ethylamino)-1-pyrrolidinyl]-6,8-difluor-1,4-dihydro-4-oxo-3-chinolincarbonsäure;

1-Cyclopropyl-6,8-difluor-1,4-dihydro-7-[3-[[(1-methylethyl)amino]methyl]-1-pyrrolidinyl]-4-oxo-3-chinolincarbonsäure;

7-(2,5-Diazabicyclo[2.2.2]oct-2-yl)-1-cyclopropyl-6,8-difluor-1,4-dihydro-4-oxo-3-chinolincarbonsäure;

7-[3-(exo-Amino)-8-azabicyclo[3.2.1]oct-8-yl]-1-cyclopropyl-6,8-difluor-1,4-dihydro-4-oxo-3-chinolincarbonsäure;

7-(1,4-Diazabicyclo[3.2.1]oct-4-yl)-1-cyclopropyl-6,8-difluor-1,4-dihydro-4-oxo-3-chinolincarbonsäure;

1-Cyclopropyl-6,8-difluor-1,4-dihydro-7-(5-methyl-2,5-diazabicyclo[2.2.1]hept-2-yl)-4-oxo-3-chinolincarbonsäure;

1-Cyclopropyl-7-(2,5-diazabicyclo[2.2.1]hept-2-yl)-6,8-difluor-1,4-dihydro-4-oxo-3-chinolincarbonsäure; oder

1-Ethyl-7-[3-[(ethylamino)methyl]-1-pyrrolidinyl]-6,8-difluor-1,4-dihydro-4-oxo-3-chinolincarbonsäure.

**Revendications**

1. Procédé pour la préparation de composés de formule:

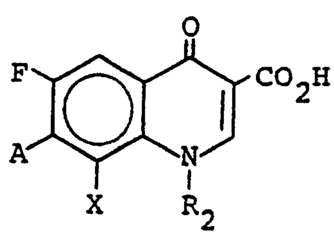

(I)

et de leurs sels basiques ou de leurs additions avec un acide pharmaceutiquement acceptables, où A est un groupe amino substitué choisi parmi (i) les groupes mono- ou dialkylamino dans lesquels le fragment alkyle possède de un à quatre atomes de carbone en chaîne droite ou ramifiée, le fragment alkyle étant facultativement substitué par un ou plusieurs radicaux hydroxy, amino, méthylamino ou diméthylamino; (ii) les groupes amino hétérocycliques à cinq ou six chaînons, les noyaux étant facultativement interrompus par un autre hétéroatome et étant facultativement substitués par un ou plusieurs radicaux alkyle ayant de un à trois atomes de carbone, hydroxy, alkoxy ayant de un à trois atomes de carbone, amino, méthylamino, éthylamino, aminométhyle, aminoéthyle, alkylaminoéthyle où le groupe alkyle possède de un à trois atomes de carbone, ou alkylaminométhyle où le groupe alkyle possède de un à trois atomes de carbone; (iii) les groupes de formule

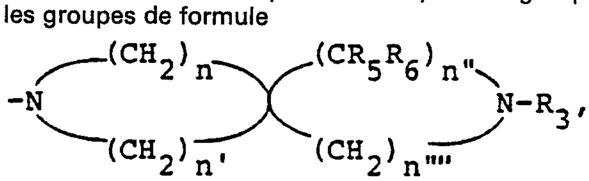

ou

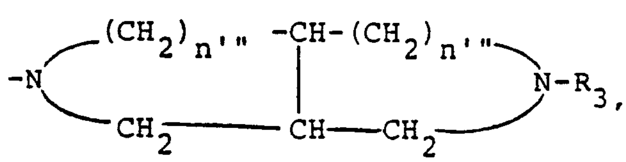

dans lesquels $R_3$ est un hydrogène, un radical alkyle ayant de un à quatre atomes de carbone ou cycloalkyle ayant de trois à six atomes de carbone, n vaut 1, 2, 3 ou 4; n' vaut 1, 2, 3 ou 4; n + n' vaut au total 2, 3, 4 ou 5; n'' vaut 0, 1 ou 2; n''' vaut 1 ou 2; n'''' vaut 0, 1 ou 2; et (iv) un groupe amino bicyclique; où X est un hydrogène ou le fluor; et où $R_2$ est un radical alkyle ayant de un à trois atomes de carbone ou cycloalkyle ayant de trois à six atomes de carbone, ce procédé consistant:

(a) à faire réagir 1,0—3,0 équivalents d'un métal alcalin nucléophile dans un solvant inerte anhydre avec un composé de formule

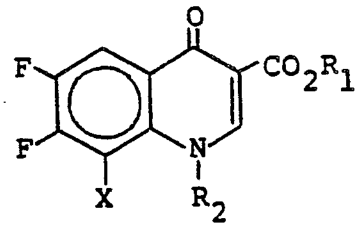

(II)

dans laquelle $R_2$ et X sont tels que définis ci-dessus, et $R_1$ est un radical alkyle ayant de un à trois atomes de carbone, à 0—150°C pour former l'un de ses sels de métaux alcalins;

(b) à ajouter au moins un équivalent de l'amine appropriée au sel de métal alcalin dans un solvant aprotique ou in situ à la solution du sel de métal alcalin contenant un cosolvant aprotique, et à chauffer le mélange réactionnel entre 60 et 120°C jusqu'à ce que la réaction soit terminée;

(c) à évaporer le solvant et à traiter le solide avec un ester, puis avec un acide dilué jusqu'à pH 5,5—7,5; et

(d) si on le souhaite, à convertir le composé obtenu de formule I en l'un de ses sels basiques ou d'addition avec un acide pharmaceutiquement acceptables;

les étapes (a), (b) et (c) du procédé étant mises en oeuvre dans un procédé en un pot.

2. Procédé selon la revendication 1, dans lequel, dans l'étape (a), le solvant inerte anhydre est choisi parmi le méthanol, l'éthanol, l'isopropanol, le dioxanne, le tétrahydrofuranne, le diglyme, la pyridine, la collidine, le dichlorométhane ou le chloroforme.

3. Procédé selon la revendication 1 ou 2, dans lequel on utilise comme matière de départ l'ester méthylique ou éthylique de l'acide 1-éthyl- ou 1-cyclopropyl-6,7,8-trifluoro-1,4-dihydro-4-oxo-3-quinoléine-carboxylique.

4. Procédé selon la revendication 1, 2 ou 3, dans lequel le nucléophile de métal alcalin est un hydroxyde, halogénure ou triméthylsilanoate de métal alcalin.

5. Procédé selon la revendication 4, dans lequel l'hydroxyde de métal alcalin est l'hydroxyde de sodium ou de potassium.

6. Procédé selon la revendication 4, dans lequel le nucléophile de métal alcalin est l'iodure de lithium, le bromure de lithium, le triméthylsilyloxyde de sodium ou le triméthylsilanoate de potassium.

7. Procédé selon la revendication 1, dans lequel, dans l'étape (b), le solvant aprotique est choisi parmi le N,N-diméthylformamide, le N,N-diméthylacétamide, le diméthylsulfoxyde et l'acétonitrile.

8. Procédé selon l'une quelconque des revendications précédentes, dans lequel A est ledit groupe amino hétérocyclique à cinq ou six chaînons, et où ledit autre hétéroatome est choisi parmi l'oxygène, le soufre, —SO—, —$SO_2$ et N—$R_3$, où $R_3$ est tel que défini dans la revendication 1.

9. Procédé selon l'une quelconque des revendications 1 à 7, dans lequel A est ledit groupe amino hétérocyclique à cinq ou six chaînons, lequel noyau est facultativement interrompu par N—$R_3$, et ledit noyau est facultativement substitué par un radical alkyle ayant de un à trois atomes de carbone, amino, méthylamino, éthylamino, alkylaminoéthyle où le fragment alkyle possède de un à trois atomes de carbone, ou alkylaminométhyle où le fragment alkyle a de un à trois atomes de carbone, où $R_3$ est un hydrogène ou un radical alkyle ayant de un à quatre atomes de carbone.

10. Procédé selon l'une quelconque des revendications 1 à 7, dans lequel A est la pipérazine, la N-méthylpipérazine ou une pyrrolidine ayant la formule suivante

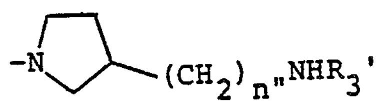

dans laquelle n'' vaut 0 ou 1 et $R'_3$ est un hydrogène ou le radical méthyle, éthyle ou 1- ou 2-propyle.

11. Procédé selon l'une quelconque des revendications 1 à 7, où A a la formule suivante

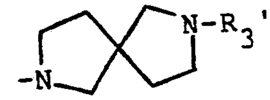

dans laquelle $R'_3$ est un hydrogène ou le radical méthyle, éthyle ou 1- ou 2-propyle.

12. Procédé selon l'une quelconque des revendications 1 à 7, dans lequel A a l'une des formules suivantes

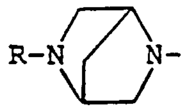 , 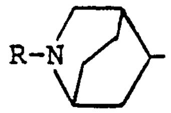 , 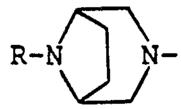 , 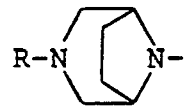 ,

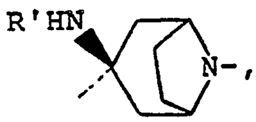 , 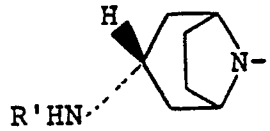 ou 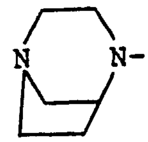

où R est un hydrogène ou le radical méthyle, éthyle, 1- ou 2-propyle, hydroxyéthyle, benzyle ou p-aminobenzyle, et R' est un hydrogène ou le radical acétyle.

13. Procédé selon l'une quelconque des revendications 1 à 7, dans lequel on prépare l'un des composés suivants:

acide 7-[3-(aminométhyl)-1-pyrrolidinyl]-1-cyclopropyl-6,8-difluoro-1,4-dihydro-4-oxo-3-quinoléinecarboxylique;

acide 1-cyclopropyl-7-[3-[(éthylamino)méthyl]-1-pyrrolidinyl]-6,8-difluoro-1,4-dihydro-4-oxo-3-quinoléinecarboxylique;

acide 7-[3-amino-1-pyrrolidinyl]-1-cyclopropyl-6,8-difluoro-1,4-dihydro-4-oxo-3-quinoléine-carboxylique;

acide 1-cyclopropyl-6,8-difluoro-1,4-dihydro-7-[3-[(méthylamino)méthyl]-1-pyrrolidinyl]-4-oxo-3-quinoléinecarboxylique;

acide 1-cyclopropyl-7-[3-(éthylamino)-1-pyrrolidinyl]-6,8-difluoro-1,4-dihydro-4-oxo-3-quinoléinecarboxylique;

acide 1-cyclopropyl-6,8-difluoro-1,4-dihydro-7-[3-[[(1-méthyléthyl)amino]méthyl]-1-pyrrolidinyl]-4-oxo-3-quinoléinecarboxylique;

acide 7-(2,5-diazabicyclo[2.2.2]oct-2-yl)-1-cyclopropyl-6,8-difluoro-1,4-dihydro-4-oxo-3-quinoléine-carboxylique;

acide 7-[3-(exo-amino)-8-azabicyclo[3.2.1]oct-8-yl]-1-cyclopropyl-6,8-difluoro-1,4-dihydro-4-oxo-3-quinoléinecarboxylique;

acide 7-(1,4-diazabicyclo[3.2.1]oct-4-yl)-1-cyclopropyl-6,8-difluoro-1,4-dihydro-4-oxo-3-quinoléine-carboxylique;

acide 1-cyclopropyl-6,8-difluoro-1,4-dihydro-7-(6-méthyl-2,5-diazabicyclo[2.2.1]hept-2-yl]-4-oxo-3-quinoléinecarboxylique;

acide 1-cyclopropyl-7-[2,5-diazabicyclo[2.2.1]hept-2-yl]-6,8-difluoro-1,4-dihydro-4-oxo-3-quinoléine-carboxylique; ou

acide 1-éthyl-7-[3-[(éthylamino)méthyl]-1-pyrrolidinyl]-6,8-difluoro-1,4-dihydro-4-oxo-3-quinoléine-carboxylique.